# EUROPÄISCHE PATENTSCHRIFT

(11) **EP 0 777 663 B1**
(45) Veröffentlichungstag und Bekanntmachung des Hinweises auf die Patenterteilung: **26.05.1999**
(21) Anmeldenummer: 95929833.2
(22) Anmeldetag: 10.08.1995
(51) Int. Cl.: C07D 309/32, C07D 405/04, A61K 31/35

(54) **VERWENDUNG VON SUBSTITUIERTEN 6-AMINO-4H-PYRANEN**
USE OF SUBSTITUTED 6-AMINO-4H-PYRANS
UTILISATION DE 6-AMINO-4H-PYRANNES SUBSTITUES

(30) Priorität: 23.08.1994 DE 4429786
(43) Veröffentlichungstag der Anmeldung: 11.06.1997
(73) Patentinhaber: BAYER AG, 51368 Leverkusen (DE)
(72) Erfinder: URBAHNS, Klaus, D-42115 Wuppertal (DE); HEINE, Hans-Georg, D-47800 Krefeld (DE); JUNGE, Bodo, D-42399 Wuppertal (DE); SCHOHE-LOOP, Rudolf, D-42327 Wuppertal (DE); SOMMERMEYER, Henning, D-51061 Köln (DE); GLASER, Thomas, D-51491 Overrath (DE); WITTKA, Reilinde, D-51069 Köln (DE); DE VRY, Jean-Marie-Viktor, D-51503 Rösrath (DE)
(86) Internationale Anmeldenummer: EP9503168
(87) Internationale Veröffentlichungsnummer: WO9606091

(56) Entgegenhaltungen:
- EP-A- 0 088 276
- DE-A- 2 235 406
- CHEMICAL ABSTRACTS, vol. 101, no. 3, 1984, Columbus, Ohio, US; abstract no. 23271g, SOTO,JOSE 'SYNTHESIS OF HETEROCYCLIC COMPOUNDS.XXXVI.' Seite 582 ; & HETEROCYCLES, Bd.22, Nr.1, 1984, JAPAN Seiten 1 - 6
- CHEMICAL ABSTRACTS, vol. 113, no. 28, 1990, Columbus, Ohio, US; abstract no. 78235b, ABDEL-LATIF,F. 'SYNTHESIS OF HETEROCYCLES THROUGH REACTIONS OF NUCLEOPHILES WITH ACRYLONITRILES.' Seite 769 ; & INDIAN J. CHEM. SECT B, Bd.29B, Nr.4, 1990, INDIA Seiten 322 - 325
- CHEMICAL ABSTRACTS, vol. 97, no. 3, 1982, Columbus, Ohio, US; abstract no. 23581g, SHARANIN YU. 'NITRILE CYCLISATION REACTIONS.IV.' Seite 692 ; & ZH. ORG. KHIM., Bd.18, Nr.3, 1982, RUSS Seiten 625 - 629
- CHEMICAL ABSTRACTS, vol. 92, no. 27, 1980, Columbus, Ohio, US; abstract no. 94180h, ABRAMENKO,YU ET AL. 'SIMPLE METHODS FOR PREPARING 2-AMINO-3-CYANO-4H-PYRANS.' Seite 571 ; & NOV. KHIM. SREDTSTVA ZASHCH. RAST., 1979, RUSS Seiten 7 - 11

## Beschreibung

Die vorliegende Erfindung betrifft die Verwendung von teilweise bekannten substituierten 6-Amino-4H-pyranen als Arzneimittel, neue Wirkstoffe, ein Verfahren zu ihrer Herstellung, insbesondere ihre Verwendung als cerebral wirksame Mittel.

2-Amino-3-cyano-4H-pyrane sind aus einigen Publikationen bereits bekannt [vgl. hierzu Magn. Reson. Chem. 23 (9), 793-4, 1985; Nor. Khim. Sredstva Zashch. Rast- 7-11, 1979].

In EP-A-0 088 276 und DE-A-2 235 408 werden substituierte 2-Amino-3-alkoxycarbonyl-pyrane mit spasmolytischer Wirkung in den Gefäßen des zentralen Nervensystems beschrieben. Aus einer synthetischen Publikation (Heterocycles 1984,22,1-6) sind substituierte 2-Amino-3-cyano-pyrane bekannt.

Es wurde nun gefunden, daß die substituierten 6-Amino-4H-pyrane der allgemeinen Formel (I), 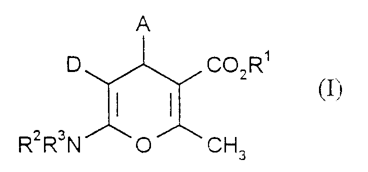 in welcher
- A: für Phenyl oder Pyridyl steht, die gegebenenfalls bis zu 2-fach gleich oder verschieden durch Nitro, Cyano, Fluor, Chlor, Brom, Iod, Trifluormethyl, Methoxy, Phenyl oder durch Methylthio substituiert sind,
- D: für Cyano steht,
- R¹: für Wasserstoff oder für geradkettiges oder verzweigtes Alkyl mit bis zu 4 Kohlenstoffatomen steht,
- R² und R³: gleich oder verschieden sind und für Wasserstoff oder für geradkettiges oder verzweigtes Alkyl oder Acyl mit jeweils bis zu 3 Kohlenstoffatomen stehen
und deren Salze
überraschenderweise eine modulierende Wirkung auf Kaliumkanäle besitzen und somit geeignet sind zur Verwendung bei der Bekämpfung von cerebralen Erkrankungen und der Sichelzellenanämie.

Im Rahmen der Erfindung sind physiologisch unbedenkliche Salze bevorzugt. Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Die erfindungsgemäßen Verbindungen der allgemeinen Formel (I) zeigen ein nicht vorhersehbares, wertvolles pharmakologisches Wirkspektrum.

Sie sind Kanalmodulatoren mit Selektivität für Calciumabhängige Kalium-Kanäle großer Leitfähigkeit (BK(Ca)-Kanäle), insbesondere des zentralen Nervensystems.

Aufgrund ihrer pharmakologischen Eigenschaften können sie für die Herstellung von Arzneimitteln zur Behandlung von zentral degenerativen Erkrankungen eingesetzt werden, wie bei Auftreten von Demenzen wie Multiinfarktdemenz (MID), primär degenerativer Demenz (PDD), präseniler und seniler Demenz bei Alzheimerscher Krankheit, HIV-Demenz und andere Demenzformen. Ferner eignen sie sich zur Behandlung von Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe und Behandlung, und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und bei der Bekämpfung von cerebralen Erkrankungen.

Die erfindungsgemäßen Verbindungen der allgemeinen Formel (I) zeigen ein nicht vorhersehbares, wertvolles pharmakologisches Wirkspektrum.

Sie sind Kanalmodulatoren mit Selektivität für Calciumabhängige Kalium-Kanäle großer Leitfähigkeit (BK(Ca)-Kanäle), insbesondere des zentralen Nervensystems.

Aufgrund ihrer pharmakologischen Eigenschaften können sie für die Herstellung von Arzneimitteln zur Behandlung von zentral degenerativen Erkrankungen eingesetzt werden, wie bei Auftreten von Demenzen wie Multiinfarktdemenz (MID), primär degenerativer Demenz (PDD), präseniler und seniler Demenz bei Alzheimerscher Krankheit, HIV-Demenz und andere Demenzformen. Ferner eignen sie sich zur Behandlung von Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe und Behandlung, und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Bluthochdruck, Arrhythmie, Angina und Diabetes.

Die Erfindung betrifft außerdem neue Verbindungen der Formel (I) 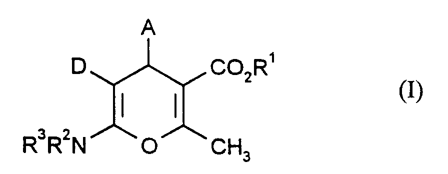 und deren Salze,
mit den in der folgenden Tabelle angegebenen Substituentenbedeutungen:

| **A** | **D** | **R**^{**1**} | **R**^{**2**} | **R**^{**3**} |
|---|---|---|---|---|
| C₆H₅ | CN | CH₃ | H | H |
| C₆H₄-o-CF₃ | CN | CH₃ | H | H |
| C₆H₄-m-NO₂ | CN | CH₃ | H | H |
| C₆H₃-o,m-Cl | CN | CH₃ | H | H |
| C₆H₄-p-CF₃ | CN | CH₃ | H | H |
| C₆H₄-p-Cl | CN | CH₃ | H | H |
| C₆H₃-o,m-Cl | CN | C₂H₅ | H | H |
| C₅H₄-p-OCH₃ | CN | CH₃ | H | H |
| C₆H₄-p-C₆H₅ | CN | CH₃ | H | H |
| C₆H₃-m,p-Cl | CN | CH₃ | H | H |

Die Verbindungen der allgemeinen Formel (I) werden hergestellt, indem man

### [A] im Fall, daß D = Cyano

Verbindungen der allgemeinen Formel (II) 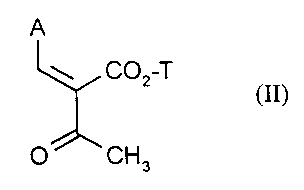 in welcher
- A: den angegebenen Bedeutungsumfang umfaßt
und
- T: den oben angegebenen Bedeutungsumfang von R¹ bat, aber nicht für Wasserstoff steht,
mit Malodinitril, in organischen Lösemitteln und in Anwesenheit einer Base,
umsetzt,
oder

### [B] Verbindungen der allgemeinen Formel (II) mit Verbindungen der allgemeinen Formel (III)

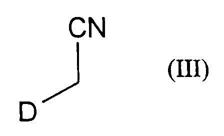

in welcher
- D, R² und R³: die angegebene Bedeutung haben
umsetzt.

Das Verfahren kann durch folgendes Formelschema erläutert werden:

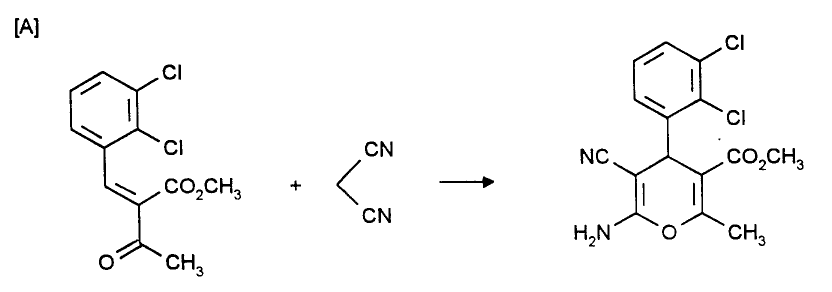

Als Lösemittel eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol, oder Pyridin. Ebenso ist es möglich, Gemische der genannten Lösemittel zu verwenden. Besonders bevorzugt ist Methanol.

Als Basen eignen sich im allgemeinen Alkalihydride- oder alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt ist Triethylamin.

Die Base wird im allgemeinen in einer Menge von 1 mol bis 5 mol, bevorzugt von 1 mol bis 2 mol, bezogen auf 1 mol der Verbindungen der allgemeinen Formel (II) eingesetzt.

Die Umsetzung mit Verbindungen der allgemeinen Formel (III) erfolgt im allgemeinen in einem Temperaturbereich von 0°C bis 150°C, bevorzugt von 40°C bis 80°C.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Enantiomerenreine Formen erhält man z.B., indem man Diastereomerengemische der Verbindungen der allgemeinen Formel (I), in welcher R¹ für einen optisch aktiven Esterrest steht, nach üblicher Methode trennt, anschließend entweder direkt umestert oder zuerst die chiralen Carbonsäuren herstellt und dann durch Veresterung die enantiomerenreinen Verbindungen herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

### ⁸⁶Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt. Dazu werden Ratten C6-BU1-Glioma-Zellen verwendet. Aus den durch Flüssigkeitszintillation gewonnenen Daten wird die durch Ionomycin hervorgerufene Erhöhung des Rb-Effluxes über den Basalefflux berechnet und als 100 % gesetzt. Die Stimulationen in Gegenwart von Prüfsubstanzen werden dann auf diesen Wert bezogen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formeln (I) / (Ia) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Alt und Schwere der Erkrankung, der Art der Zubereitung und Applikation, sowie dem Zeitpunkt bzw. Intervall, zu welchem die Verabreichung erfolgt.

### Laufmittelgemische:

a) : Toluol / Essigsäureethylester 1:1
b) : Toluol / Essigsäureethylester 3:1

### Herstellungsbeispiele

### Beispiel 1

6-Amino-5-cyan-4-(2,3-dichlorphenyl)-2-methyl-4H-pyran-3-carbonsäuremethylester 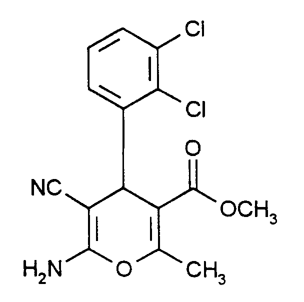 1,8 g (28 mmol) Malodinitril, 7,1g (25 mmol) 2-Acetyl-3-(2,3-dichlorphenyl)acrylsäuremethylester, 0,3g (3 mmol) Triethylamin und 0,1 g (2,4 mmol) Eisessig werden in 50 ml Methanol zum Rückfluß erhitzt. Nach 10 min. fällt ein kristalliner Niederschlag aus. Man fügt 60 ml Methanol zu und erhitzt die Suspension weiter zum Rückfluß. Nach dem Abkühlen auf Raumtemperatur wird der Niederschlag abgesaugt und mit kaltem Methanol gewaschen. Man erhält 7,2 g (85%) vom Smp. 192-194.

In Analogie zur Vorschrift des Beispiels 1 werden die in Tabelle 1 aufgeführten Verbindungen hergestellt:

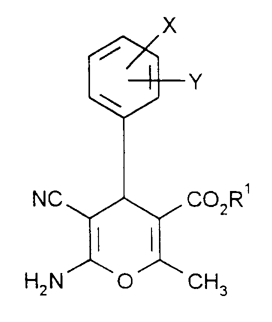

| **Bsp.-Nr.** | **X, Y** | **R**^{**1**} | **Ausbeute (% d.Th.)** | **Fp. (°C)** | **R**_{**f**} ***** |
|---|---|---|---|---|---|
| 2 | 2-H, 3-H | CH₃ | 15 | 170-4 | 0,46 / a |
| 3 | 2-CF₃, 3-H | CH₃ | 64 | 171-4 | 0,61 / a |
| 4 | 3-NO₂, 2-H | CH₃ | 46 | 195-9 | 0,28 / b |
| 5 | 4-CF₃, 3-H | CH₃ | 57 | 152-5 | 0,49 / a |
| 6 | 4-Cl, 3-H | CH₃ | 15 | 153-4 | 0,45 / a |
| 7 | 2-Cl, 3-Cl | C₂H₅ | 25 | 167-71 | |
| 8 | 4-OCH₃, 3-H | CH₃ | 19 | 136-40 | |
| 9 | 4-C₆H₅ | CH₃ | 28 | 177-79 | |
| 10 | 3-Cl, 4-Cl | CH₃ | 25 | 167-70 | |

## Patentansprüche

1. Verwendung von substituierten 6-Amino-4H-pyranen der allgemeinen Formel (I) 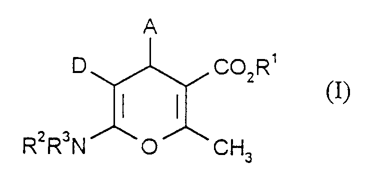 in welcher
A für Phenyl oder Pyridyl steht, die gegebenenfalls bis zu 2-fach gleich oder verschieden durch Nitro, Cyano, Fluor, Chlor, Brom, Iod, Trifluormethyl, Methoxy, Phenyl oder durch Methylthio substituiert sind,
D für Cyano steht,
R¹ für Wasserstoff oder für geradkettiges oder verzweigtes Alkyl mit bis zu 4 Kohlenstoffatomen steht,
R² und R³ gleich oder verschieden sind und
für Wasserstoff oder für geradkettiges oder verzweigtes Alkyl oder Acyl mit jeweils bis zu 3 Kohlenstoffatomen stehen
und deren Salze
zur Herstellung von Arzneimitteln zur Behandlung von cerebralen Erkrankungen, die durch Modulierung des Kaliumkanals behandelbar sind.

2. Verwendung nach Anspruch 1 zur Herstellung von Arzneimitteln zur Behandlung von zentral degenerativen Erkrankungen.

3. Verwendung nach Anspruch 1 zur Herstellung von Medikamenten zur Behandlung von Demenzen.

4. Verwendung nach Anspruch 1 zur Herstellung von Arzneimitteln zur Behandlung von Hirnleistungsstörungen im Alter.

5. Verwendung nach Anspruch 1 zur Herstellung von Arzneimitteln zur Behandlung von Folgen cerebraler Durchblutungsstörungen.

6. Substituierte 6-Amino-4H-pyrane der Reihe
6-Amino-5-cyano-4-phenyl-2-methyl-4H-pyran-3-carbonsäuremethylester
6-Amino-5-cyano-4-(2-trifluormethyl-phenyl)-2-methyl-4H-pyran-3-carbonsäuremethylester
6-Amino-5-cyano-4-(3-nitro-phenyl)-2-methyl-4H-pyran-3-carbonsäuremethylester
6-Amino-5-cyano-4-(2,3-dichlor-phenyl)-2-methyl-4H-pyran-3-carbonsäuremethylester
6-Amino-5-cyano-4-(4-trifluormethyl-phenyl)-2-methyl-4H-pyran-3-carbonsäuremethylester
6-Amino-5-cyano-4-(4-chlor-phenyl)-2-methyl-4H-pyran-3-carbonsäuremethylester
6-Amino-5-cyano-4-(2,3-dichlor-phenyl)-2-methyl-4H-pyran-3-carbonsäureethylester
6-Amino-5-cyano-4-(4-methoxy-phenyl)-2-methyl-4H-pyran-3-carbonsäuremethylester
6-Amino-5-cyano-4-(4-phenyl-phenyl)-2-methyl-4H-pyran-3-carbonsäuremethylester
6-Amino-5-cyano-4-(3,4-dichlor-phenyl)-2-methyl-4H-pyran-3-carbonsäuremethylester.

7. Verfahren zur Herstellung von substituierten 6-Amino-4H-pyranen nach Anspruch 6, dadurch gekennzeichnet, daß man die entsprechenden Ylidenverbindungen mit Malondinitril in organischen Lösemitteln in Anwesenheit einer Base umsetzt.

8. Arzneimittel enthaltend substituiertes 6-Amino-4H-pyran nach Anspruch 6 sowie übliche Formulierungshilfsmittel.

## Claims

1. Use of substituted 6-amino-4H-pyrans of the general formula (I) 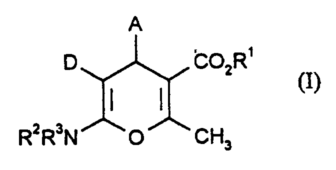 in which
A represents phenyl or pyridyl, each of which is optionally substituted up to 2 times by identical or different substituents from the group consisting of nitro, cyano, fluorine, chlorine, bromine, iodine, trifluoromethyl, methoxy and phenyl or methylthio,
D represents cyano,
R¹ represents hydrogen or straight-chain or branched alkyl having up to 4 carbon atoms,
R² and R³ are identical or different and represent hydrogen or straight-chain or branched alkyl or acyl in each case having up to 3 carbon atoms,
and their salts,
for the production of medicaments for the treatment of cerebral disorders which can be treated by modulation of the potassium channel.

2. Use according to Claim 1 for the production of medicaments for the treatment of degenerative central nervous system disorders.

3. Use according to Claim 1 for the production of medicaments for the treatment of dementias.

4. Use according to Claim 1 for the production of medicaments for the treatment of brain function disorders in old age.

5. Use according to Claim 1 for the production of medicaments for the treatment of sequelae of cerebral circulatory disorders.

6. Substituted 6-amino-4H-pyrans of the series
Methyl 6-amino-5-cyano-4-phenyl-2-methyl-4H-pyran-3-carboxylate
Methyl 6-amino-5-cyano-4-(2-trifluoromethyl-phenyl)-2-methyl-4H-pyran-3-carboxylate
Methyl 6-amino-5-cyano-4-(3-nitro-phenyl)-2-methyl-4H-pyran-3-carboxylate
Methyl 6-amino-5-cyano-4-(2,3-dichloro-phenyl)-2-methyl-4H-pyran-3-carboxylate
Methyl 6-amino-5-cyano-4-(4-trifluoromethyl-phenyl)-2-methyl-4H-pyran-3-carboxylate
Methyl 6-amino-5-cyano-4-(4-chloro-phenyl)-2-methyl-4H-pyran-3-carboxylate
Ethyl 6-amino-5-cyano-4-(2,3-dichloro-phenyl)-2-methyl-4H-pyran-3-carboxylate
Methyl 6-amino-5-cyano-4-(4-methoxy-phenyl)-2-methyl-4H-pyran-3-carboxylate
Methyl 6-amino-5-cyano-4-(4-phenyl-phenyl)-2-methyl-4H-pyran-3-carboxylate
Methyl 6-amino-5-cyano-4-(3,4-dichloro-phenyl)-2-methyl-4H-pyran-3-carboxylate.

7. Process for the preparation of substituted 6-amino-4H-pyrans according to Claim 6, characterized in that the corresponding ylidene compounds are reacted with malononitrile in organic solvents in the presence of a base.

8. Medicaments containing substituted 6-amino-4H-pyran according to Claim 6 and customary formulation auxiliaries.

## Revendications

1. Utilisation de 6-amino-4H-pyrannes substitués répondant à la formule générale (I) 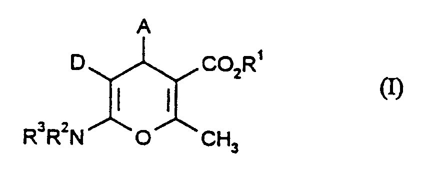 dans laquelle
A représente un groupe phényle ou un groupe pyridyle qui portent, le cas échéant, jusqu'à deux substituants identiques ou différents nitro, cyano, fluoro, chloro, bromo, iodo, trifluorométhyle, méthoxy, phényle ou méthylthio,
D représente un groupe cyano,
R¹ représente un atome d'hydrogène ou un groupe alkyle à chaîne droite ou ramifiée contenant jusqu'à 4 atomes de carbone,
R² et R³ sont identiques ou différents et représentent un atome d'hydrogène ou bien un groupe alkyle ou un groupe acyle à chaîne droite ou ramifiée contenant respectivement jusqu'à 3 atomes de carbone,
et de leurs sels,
pour la préparation de médicaments pour le traitement de maladies cérébrales qui peuvent être traitées par modulation du canal du potassium.

2. Utilisation selon la revendication 1 pour la préparation de médicaments pour le traitement de maladies dégénératives du système nerveux central.

3. Utilisation selon la revendication 1 pour la préparation de médicaments pour le traitement de démences.

4. Utilisation selon la revendication 1 pour la préparation de médicaments pour le traitement de troubles des performances cérébrales dues à l'âge.

5. Utilisation selon la revendication 1 pour la préparation de médicaments pour le traitement de suites de troubles d'hémorragies cérébrales.

6. 6-amino-4H-pyrannes substitués de la série comprenant:
l'ester méthylique de l'acide 6-amino-5-cyano-4-phényl-2-méthyl-4H-pyranne-3-carboxylique,
l'ester méthylique de l'acide 6-amino-5-cyano-4-(2-trifluorométhyl-phényl)-2-méthyl-4H-pyranne-3-carboxylique,
l'ester méthylique de l'acide 6-amino-5-cyano-4-(3-nitro-phényl)-2-méthyl-4H-pyranne-3-carboxylique,
l'ester méthylique de l'acide 6-amino-5-cyano-4-(2,3-dichloro-phényl)-2-méthyl-4H-pyranne-3-carboxylique,
l'ester méthylique de l'acide 6-amino-5-cyano-4-(4-trifluorométhyl-phényl)-2-méthyl-4H-pyranne-3-carboxylique,
l'ester méthylique de l'acide 6-amino-5-cyano-4-(4-chloro-phényl)-2-méthyl-4H-pyranne-3-carboxylique,
l'ester éthylique de l'acide 6-amino-5-cyano-4-(2,3-dichloro-phényl)-2-méthyl-4H-pyranne-3-carboxylique,
l'ester méthylique de l'acide 6-amino-5-cyano-4-(4-méthoxy-phényl)-2-méthyl-4H-pyranne-3-carboxylique,
l'ester méthylique de l'acide 6-amino-5-cyano-4-(4-phényl-phényl)-2-méthyl-4H-pyranne-3-carboxylique,
l'ester méthylique de l'acide 6-amino-5-cyano-4-(3,4-dichloro-phényl)-2-méthyl-4H-pyranne-3-carboxylique.

7. Procédé pour la préparation de 6-amino-4H-pyrannes substitués selon la revendication 6, caractérisé en ce qu'on fait réagir les composés d'ylidène correspondants avec du malonitrile dans des solvants organiques en présence d'une base.

8. Médicament contenant du 6-amino-4H-pyranne substitué selon la revendication 6, ainsi que des adjuvants de formulation habituels.
